# EUROPEAN PATENT SPECIFICATION

(11) **EP 0 717 746 B1**
(45) Date of publication and mention of the grant of the patent: **15.07.1998**
(21) Application number: 94927522.6
(22) Date of filing: 29.08.1994
(51) Int. Cl.: C07F 9/30, A61K 31/66, C07F 9/32, C07F 9/48

(54) **PHOSPHINIC ACID DERIVATIVES WITH ANTI-HYPER GLYCEMIC AND/OR ANTI-OBESITY ACTIVITY**
PHOSPHINSÄURE-DERIVATE MIT ANTI-HYPERGLYZEMIE UND/ODER ANTI-FETTSUCHT WIRKUNG
DERIVES D'ACIDE PHOSPHINIQUE AYANT UNE ACTION CONTRE L'HYPERGLYCEMIE ET/OU CONTRE L'OBESITE

(30) Priority: 09.09.1993 GB 9318744; 22.11.1993 GB 9323981; 08.02.1994 GB 9402341
(43) Date of publication of application: 26.06.1996
(73) Proprietor: SMITHKLINE BEECHAM PLC, Brentford, Middlesex TW8 9EP (GB)
(72) Inventor: BEELEY, Lee, James, SmithKline Beecham Phar., Epsom, Surrey KT18 5XQ (GB); BERGE, John, Michael, SmithKline Beecham Phar., Epsom, Surrey KT18 5XQ (GB)
(74) Representative: Rutter, Keith, Dr.
(86) International application number: EP9402886
(87) International publication number: WO9507284

(56) References cited:
- EP-A- 0 262 785
- WO-A-94/02493
- DE-A- 3 630 903

## Description

This invention relates to novel compounds, to a process for preparing such compounds, to pharmaceutical compositions containing such compounds and to the use of such compounds and compositions in medicine and agriculture.

International Patent Application, Publication Number 94/02493 discloses certain phosphonated arylethanolamine derivatives which are stated to have *inter alia* good anti-hyperglycaemic and/or anti-obesity activity.

It has been discovered that a series of phosphinated arylethanolamine derivatives show selective β₃ adrenoceptor agonist activity and hence are indicated to possess good anti-hyperglycaemic and/or anti-obesity activity with especially good selectivity from cardiac and tremorigenic side effects.

These compounds are also indicated to have potential in the treatment of gastrointestinal disorders such as peptic ulceration, oesophagitis, gastritis and duodenitis, intestinal ulcerations, including inflammatory bowel disease, and irritable bowel syndrome and also for the treatment of gastrointestinal ulcerations, especially when induced by non-steroidal anti-inflammatory drugs or corticosteroids.

These compounds also have potential as growth promoters for livestock and for decreasing birth mortality rate and increasing the post-natal survival rate in livestock. These compounds may also be of use in increasing the high-density-lipoprotein (HDL) cholesterol concentration and decreasing the triglyceride concentration in human blood serum and are therefore of potential use in the treatment and/or prophylaxis of atherosclerosis. They are also indicated to be useful for the treatment of hyperinsulinaemia.

Accordingly the present invention provides a compound of formula (I): 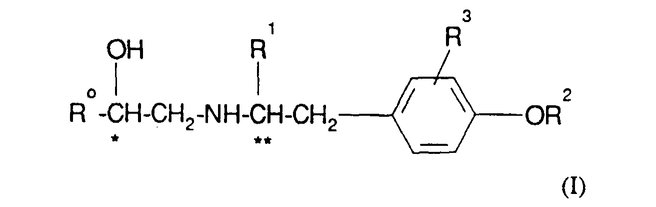 or a pharmaceutically acceptable salt thereof, or a pharmaceutically acceptable solvate thereof,
wherein,
-R^{o} represents a substituted or unsubstituted aryl group;
R¹ represents hydrogen or an alkyl group;
R² represents a moiety of formula (a): 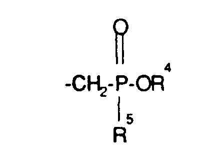 wherein R⁴ and R⁵ each independently represent hydrogen, alkyl, substituted alkyl, cycloalkyl or aryl and R³ represents hydrogen, halogen, alkyl or alkoxy.

Suitably, R^{o} is a substituted phenyl group.

Suitable substituents for R^{o} are halogen and hydroxy.

Examples of R^{o} include a 3-chlorophenyl and 3,4-dihydroxyphenyl.

Suitably, R¹ is an alkyl group.

When R¹ is alkyl, it is favourably a C₁₋₆ alkyl group, especially a methyl group.

When R⁴ and R⁵ each independently represent substituted alkyl, a preferred substituent is an hydroxy group.

Suitably, R⁴ and R⁵ each independently represent hydrogen, alkyl, phenyl, hydroxyalkyl or cycloalkyl.

Suitably, R⁵ represents alkyl, for example ethyl, or phenyl.

Suitably R⁴ is hydrogen or alkyl, for example ethyl, and R⁵ is alkyl, for example ethyl, or phenyl.

Preferably, R⁴ is hydrogen.

Suitably, R³ represents hydrogen.

The compounds of formula (I) have one or two asymmetric carbon atoms, marked with an asterisk (*) or two asterisks (**) in the formula. These compounds may therefore exist in up to four steroisomeric forms. The present invention encompasses all stereoisomers of the compounds of the general formula (I) whether free from other isomers, or admixed with other isomers in any proportion, such as mixtures of diastereoisomers and racemic mixtures of enantiomers.

Preferably, the asymmetric carbon atom indicated by a single asterisk (*) is in the R-configuration.

Preferably, the asymmetric carbon atom indicated by two asterisks (**) is in the R-configuration.

One suitable form of a compound of formula (I) is a mixture of the RR and SS enantiomers.

One suitable form of a compound of formula (I) is a mixture of RR RS epimers.

One favoured form of a compound of formula (I) is the RR enantiomer.

The term 'alkyl' when used alone or when forming part of other groups (such as the 'alkoxy' group) includes straight- or branched-chain alkyl groups containing 1 to 12 carbon atoms, suitably 1 to 6 carbon atoms, examples include methyl, ethyl, n-propyl, isopropyl, n-butyl, isobutyl or tert-butyl group.

The term 'cycloalkyl' includes C₃₋₈ cycloalkyl groups, especially C₅ or C₆ cycloalkyl groups.

When used herein the term "halogen" refers to fluorine, chlorine, bromine and iodine, preferably chlorine.

When used herein the term 'aryl' includes phenyl and naphthyl optionally substituted with up to five, preferably up to three, groups selected from halogen, alkyl, phenyl, alkoxy, haloalkyl, hydroxyalkyl, hydroxy, amino, nitro, carboxy and pharmaceutically acceptable salts, esters and amides thereof, alkoxycarbonylalkyl, alkylcarbonyloxy and alkylcarbonyl groups.

A preferred aryl group is a substituted or unsubstituted phenyl group.

Preferred optional substituents for the aryl group include up to three substituents selected from halogen, hydroxy, alkoxy, hydroxyalkyl and amino.

Substituents for alkyl groups include those mentioned for aryl groups.

Suitable pharmaceutically acceptable esters of carboxyl groups include alkyl esters, especially C₁₋₆ alkyl esters such as methyl.

Suitable pharmaceutically acceptable amides are those of formula -CONR^{s}R^{t} wherein R^{s} and R^{t} each independently represent hydrogen, alkyl or alkoxyalkyl.

Suitable pharmaceutically acceptable salts include acid addition salts, salts of carboxy groups and salts of phosphonic acid groups.

Suitable pharmaceutically acceptable acid addition salts include salts with inorganic acids such, for example, as hydrochloric acid, hydrobromic acid, orthophosphoric acid or sulphuric acid, or with organic acids such, for example as methanesulphonic acid, toluenesulphonic acid, acetic acid, propionic acid, lactic acid, citric acid, fumaric acid, malic acid, succinic acid, salicylic acid, maleic acid or acetylsalicylic acid.

Suitable pharmaceutically acceptable salts of carboxy groups or phosphinic acid groups include metal salts, such as for example aluminium, alkali metal salts such as sodium or potassium, alkaline earth metal salts such as calcium or magnesium and ammonium or substituted ammonium salts, for example those with C₁₋₆ alkylamines such as triethylamine, hydroxy-C₁₋₆ alkylamines such as 2-hydroxyethylamine, bis-(2- hydroxyethyl)-amine or tri-(2-hydroxyethyl)-amine, cycloalkylamines such as bicyclohexylamine, or with procaine, dibenzylpiperidine, N-benzyl-β-phenethylamine, dehydroabietylamine, N,N'-bisdehydroabietylamine, glucamine, N-methylglucamine or bases of the pyridine type such as pyridine, collidine or quinoline.

Suitable pharmaceutically acceptable solvates are conventional solvates, preferably hydrates.

The invention also provides a process for the preparation of a compound of formula (I), or a pharmaceutically acceptable acid addition salt or a pharmaceutically acceptable solvate thereof, which process comprises reducing a compound of formula (II): 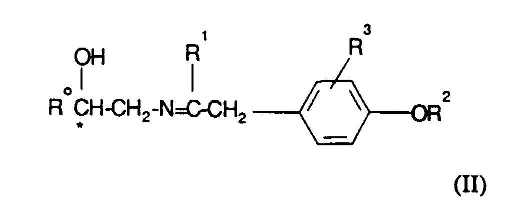 wherein R^{o}, R¹, R² and R³ are as defined in relation to formula (I),
and thereafter, if necessary, carrying out one or more of the following optional steps:
(i) converting one compound of formula (I) to another compound of formula (I); and
(ii) preparing a pharmaceutically acceptable acid addition salt of a compound of formula (I) or a pharmaceutically acceptable solvate thereof.

The reduction of the compound of formula (II) may be carried out using any suitable reduction procedure, for example by using catalytic reduction in the presence of hydrogen.

Suitable catalysts include platinum oxide and 10% palladium on charcoal.

Suitable reduction conditions include using an alkanol solvent such as methanol, at any temperature providing a convenient rate of formation of the required product, for example ambient temperature, when using a platinum oxide catalyst and at an elevated temperature such as 50°C when using a 10% palladium on charcoal catalyst, under a pressure of 1-5 atmospheres of hydrogen.

The compound of formula (II) may be prepared by reacting a compound of formula (III): 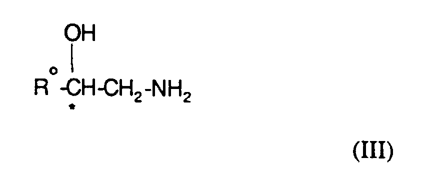 wherein R^{o} is as defined in relation to formula (I) with a compound of formula (IV): 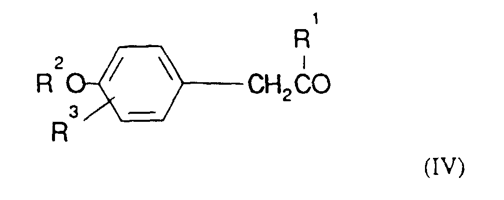 wherein R² and R³ are as defined in relation to formula (I).

The reaction between compounds of formulae (III) and (IV) may be carried out under conventional amination conditions, for example in a solvent such as methanol or toluene.

Conveniently, the compound of formula (II) is prepared in-situ by reacting a compound of the above defined formula (III) with a compound of the above defined formula (IV) under reductive amination conditions.

Suitable reductive amination conditions include reacting the compounds of formula (III) and (IV) in an alkanolic solvent, such as methanol, in the presence of a suitable reduction catalyst, such as platinum oxide, at any temperature providing a convenient rate of formation of the required product, generally ambient temperature, under hydrogen at a pressure of 1-5 atmosphere.

A compound of formula (IV) may be prepared by reducing a compound of formula (V): 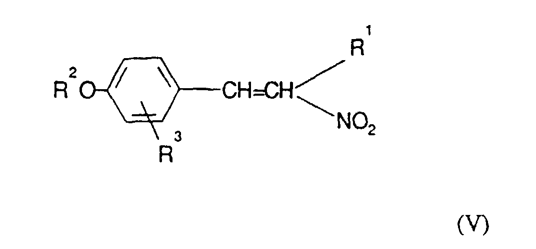 wherein R¹, R² and R³ are as defined in relation to formula (I).

The reduction of the compound of formula (V) may conveniently be carried out using iron powder in the presence of acetic acid in an aqueous solvent such as aqueous methanol, at any temperature providing a suitable rate of formation of the required product, generally at an elevated temperature and conveniently at the reflux temperature of the solvent.

A compound of formula (V) may be prepared by reacting a compound of formula (VI): 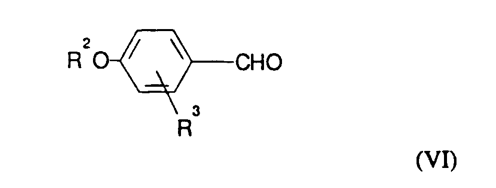 wherein, R² and R³ are as defined in relation to formula (I), with nitroalkane.

Generally, the carbon atom of the -CHO group of compound of formula (VI) is in an activated form, a suitable activated form being provided by forming an imine. of the said carbonyl group: The imine may be prepared by reacting the compound of formula (VI) with an amine, suitably a primary alkyl amine such as n-butylamine. The reaction of the compound of formula (VI) and the amine may be carried out in any suitable solvent, such as toluene, at any temperature providing a suitable rate of formation of the required product, generally at an elevated temperature such as the reflux temperature of the solvent; and preferably in the presence of a catalytic amount of toluenesulphonic acid.

The reaction between the compound of formula (VI) and nitroalkane may be carried out in glacial acetic acid, preferably in the presence of an ammonium acetate catalyst, generally at an elevated temperature such as in the range of from 60°C to 120°C, for example 100°C. When the compound of formula (VI) is in the form of an imine, the reaction with nitroalkane may be carried out in glacial acetic acid, generally at an elevated temperature such as in the range of from 60°C to 120°C, for example 100°C.

A compound of formula (VI) may be prepared from a compound of formula (VII): 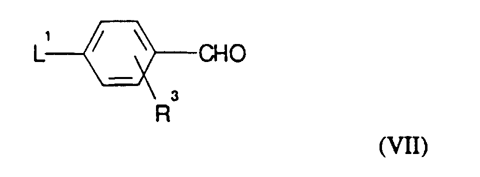 wherein R³ is as defined in relation to formula (I) and L¹ is a leaving group or atom, generally a fluorine atom, with an activated form of a compound of formula (VIII): 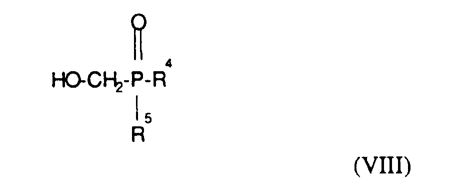 wherein R⁴ and R⁵ are as defined in relation to formula (I).

A suitable activated form of a compound of formula (VIII) is an ionic form, such as a salted form, for example an alkali metal salted form.

An activated form of a compound of formula (VIII) may be prepared by use of the appropriate conventional procedure, for example a salted form may be prepared by treating the compound of formula (VIII) with a base such as an alkali metal hydride, for example sodium hydride.

The reaction between the compounds of formulae (VII) and (VIII) may be carried out in any suitable solvent, generally an aprotic solvent such as dimethylformamide or N-methylpyrrolidinone at a low to ambient temperature, for example in the range of from -15°C to 20°C, such as 5°C.

The invention also provides a process for the preparation of a compound of formula (I), or a pharmaceutically acceptable acid addition salt or a pharmaceutically acceptable solvate thereof, which process comprises deprotecting a compound of formula (IX): 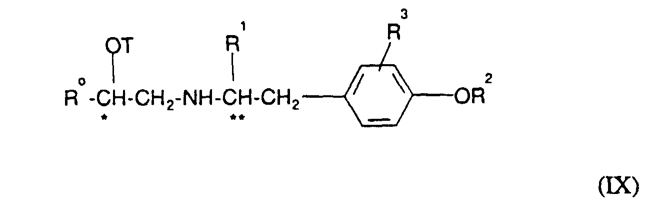 wherein R^{o}, R¹, R² and R³ are as defined in relation to formula (I) and T represents an hydroxy protecting group, and thereafter, if necessary, carrying out one or more of the following optional steps:
(i) converting one compound of formula (I) to another compound of formula (I); and
(ii) preparing a pharmaceutically acceptable acid addition salt of a compound of formula (I) or a pharmaceutically acceptable solvate thereof.

Suitable protecting groups include trialkylsilyl groups, for example a triethylsilyl group.

The deprotection of the compound of formula (IX) may be carried out using conventional methods depending upon the nature of the protecting group, for example when the protecting group is a trialkylsilyl group this may be removed by acid hydrolysis, for example by using aqueous hydrochloric acid, conveniently at ambient temperature and preferably under an inert atmosphere such as argon.

A compound of formula (IX) may be prepared by the reacting a compound of formula (X): 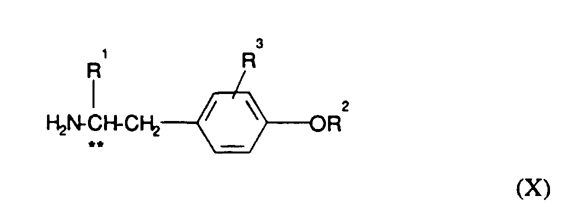 wherein R¹, R² and R³ are as defined in relation to formula (I), with a compound of formula (XI): 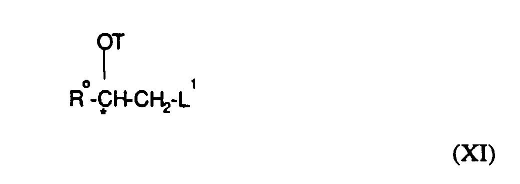 wherein R^{o} and T are as defined in relation to formula (IX) and is L¹ is a leaving group or atom, such as a halogen atom, for example an iodine atom.

The reaction between the compounds of formulae (X) and (XI) may be carried out in any suitable aprotic solvent, such as tetrahydrofuran, at any temperature providing a convenient rate of formation of the required product, generally at an elevated temperature, such as in the range of from 30°C to 120°C, and suitably at an elevated pressure, conveniently the reaction is carried out in a sealed tube using tetrahydrofuran as solvent; preferably the reaction is carried out in an inert atmosphere, generably under argon.

A compound of formula (X) wherein the **CH carbon is a chiral carbon may be prepared by the hydrogenolysis of a compound of formula (XII): 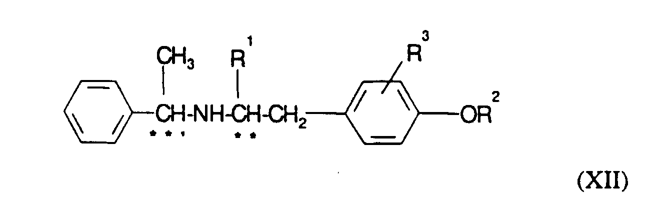 wherein R¹, R² and R³ are as defined in relation to formula (I) and the **CH carbon and ***CH carbon atoms are chiral carbon atoms.

Suitably, catalytic hydrogenolysis is used, using for example 10% palladium on charcoal in the presence of ammonium formate, suitably in an alkanolic solvent such as methanol, at any temperature providing a convenient rate of formation of the required product, for example at ambient temperature; preferably the reaction is carried out in an inert atmosphere, generably under nitrogen .

Compounds of formula (X) may also be prepared by processes analogous to those disclosed in European Patent, Publication Number 0023385.

The compound of formula (XII) may be prepared by stereoselectve reduction of a compound of formula (XIII): 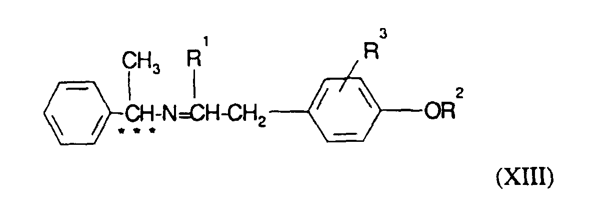 wherein R¹, R² and R³ are as defined in relation to formula (I) and the ***CH carbon is a chiral carbon.

The reduction of the compound of formula (XIII) may be carried out using catalytic reduction in the presence of hydrogen.

A preferred catalyst is platinum oxide.

Suitable reduction conditions include using an alkanol solvent such as methanol or ethanol, at any temperature providing a convenient rate of formation of the required product, conveniently at ambient temperature using a pressure of 1-5 atmospheres of hydrogen.

The compound of formula (XIII) may be prepared by reacting a compound of the above defined formula (IV) with R-α-methylbenzylamine.

The reaction between compounds of formulae (IV) and R-α-methylbenzylamine may be carried out under conventional amination conditions, for example those described above for the reaction between the compounds of formula (III) and (IV). Conveniently, as for the above defined compound of formula (II), the compound of formula (XIII) is prepared in-situ by reacting a compound of the above defined formula (IV) with R-α-methylbenzylamine under reductive amination conditions as described in relation to the compounds of formulae (III) and (IV).

A compound of formula (XI) may be prepared by protecting a compound of formula (XIV): 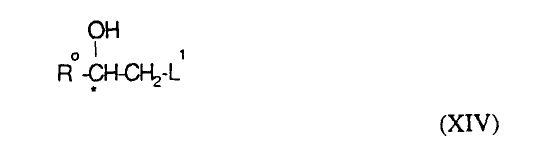 wherein R^{o} and L¹ are as defined in relation to formula (XI).

The protection of the compound of formula (XIV) may be carried out using conventional methods depending upon the nature of the protecting group required, for example for preparing a compound of formula (XIV) wherein the protecting group is a trialkylsilyl group the compound of formula (XI) is reacted with a trialkylsilyltrifluoromethanesulphonate, suitably triethylsilyltrifluoromethanesulphonate.

Suitable conversions of one compound of formula (I) into another compound of formula (I) include converting one group OR⁴ into another group OR⁴.

Suitable conversions of one group OR⁴ into another group OR⁴ include:
(i) converting OR⁴ as hydroxy into OR⁴ as alkoxy;
(ii) converting OR⁴ as alkoxy into OR⁴ as hydroxy;
(iii) converting OR⁴ as alkoxy into OR⁴ as another alkoxy group.

The abovementioned conversion (i) may be carried out under conventional phosphonate alkylation methods, using for example the appropriate alcohol (R⁴OH) in the presence of hydrogen chloride.

The abovementioned conversion (ii) may be carried out using conventional phosphonate hydrolysis methods, for example by treating the appropriate compound of formula (I) with an alkaline metal hydroxide, such as sodium hydroxide or lithium hydroxide.

The abovementioned conversion (iii) may be carried out by first converting OR⁴ as alkoxy into OR⁴ as hydroxy using the conditions set out in respect of the abovementioned conversion (ii), followed by converting the hydroxy group so formed into another alkoxy group, using the conditions set out in respect of the abovementioned conversion (i).

The abovementioned conversion (iii) is of particular use for preparing compounds of formula (I) wherein OR⁴ represents methoxy: such compounds are generally prepared from compounds of formula (I) wherein OR⁴ represents an alkyloxy group other than methoxy (suitably ethoxy) by first hydrolysing the relevant OR⁴ group (via conversion (ii)) to prepare a compound of formula (I) wherein OR⁴ represents hydroxy and thereafter methylating (via conversion (i)) to provide the required compound wherein OR⁴ represents methoxy.

The above mentioned conversions may as required be carried out at any stage in the synthetic route to the compound of formula (I), for example the conversion of OR⁴ as alkoxy to OR⁴ as hydroxy may be carried out on the compounds of formula (IV).

The protection of any reactive group or atom, may be carried out at any appropriate stage in the aforementioned processes. Suitable protecting groups include those used conventionally in the art for the particular group or atom being protected. Protecting groups may be prepared and removed using the appropriate conventional procedure. For example in the above mentioned conversion of OR⁴ as alkoxy to OR⁴ as hydroxy in a compound of formula (IV), the keto group of the compound of formula (IV) may conveniently be in a protected form, such as a ketal.

A leaving group or atom is any group or atom that will, under the reaction conditions, cleave from the starting material, thus promoting reaction at a specified site. Suitable examples of such groups unless othewise specified are halogen atoms, mesyloxy groups and tosyloxy groups.

The compounds of formulae (III) and (VIII) are known compounds or they may be prepared by processes analogous to those used to prepare known compounds, for example the compounds of formula (III) may be prepared according to methods disclosed in J.Org.Chem., 1974, 39, 914; and the compounds of formula (VIII) may be prepared according to methods disclosed in Phosphorus and Sulphur, 1978, 5, 455.

The salts, esters, amides and solvates of the compounds mentioned herein may be produced by methods conventional in the art: For example, acid addition salts may be prepared by treating a compound of formula (I) with the appropriate acid.

Esters of carboxylic acids may be prepared by conventional esterification procedures, for example alkyl esters may be prepared by treating the required carboxylic acid with the appropriate alkanol, generally under acidic conditions.

Amides may be prepared using conventional amidation procedures, for example amides of formula CONR^{s}R^{t} may be prepared by treating the relevant carboxylic acid with an amine of formula HNR^{s}R^{t,} wherein R^{S} and R^{t} are as defined above. Alternatively, a C₁₋₆ alkyl ester such as a methyl ester of the acid may be treated with an amine of the above defined formula HNR^{s}R^{t} to provide the required amide.

Compounds of formula (I) and pharmaceutically acceptable acid addition salts thereof; or a pharmaceutically acceptable solvate thereof, produced by the above processes, may be recovered by conventional methods.

If required mixtures of isomers of the compounds of the invention may be separated into individual stereoisomers by conventional means, for example by the use of an optically active acid as a resolving agent. Suitable optically active acids which maybe used as resolving agents are described in Topics in Stereochemistry', Vol. 6, Wiley Interscience, 1971, Allinger, N.L. and Eliel, W.L. Eds.

Alternatively, any enantiomer of a compound of the invention may be obtained by stereospecific synthesis using optically pure starting materials of known configuration.

The absolute configuration of compounds may be determined by conventional X-ray crystallographic techniques.

As previously indicated, the compounds of the present invention have valuable pharmacological properties:

The present invention accordingly provides a compound of formula (I) or a pharmaceutically acceptable acid addition salt thereof, or a pharmaceutically acceptable solvate thereof, for use as an active therapeutic substance.

In one aspect, the present invention provides a compound of formula (I), or a pharmaceutically acceptable acid addition salt thereof, or a pharmaceutically acceptable solvate thereof, for use in the treatment of hyperglycaemia in human or non-human animals.

The present invention further provides a compound of formula (I), or pharmaceutically acceptable acid addition salt thereof, or a pharmaceutically acceptable solvate thereof, for use in the treatment of obesity in human or non-human animals.

A compound of formula (I), or a pharmaceutically acceptable acid addition salt thereof, or a pharmaceutically acceptable solvate thereof, may be administered per se or, preferably, as a pharmaceutical composition also comprising a pharmaceutically acceptable carrier.

Accordingly, the present invention also provides a pharmaceutical composition comprising a compound of formula (I), or a pharmaceutically acceptable acid addition salt thereof, or a pharmaceutically acceptable solvate thereof, and a pharmaceutically acceptable carrier therefor.

As used herein the term "pharmaceutically acceptable" embraces compounds, compositions and ingredients for both human and veterinary use: for example the term "pharmaceutically acceptable salt" embraces a veterinarily acceptable salt.

The composition may, if desired, be in the form of a pack accompanied by written or printed instructions for use.

Usually the pharmaceutical compositions of the present invention will be adapted for oral administration, although compositions for administration by other routes, such as by injection, are also envisaged.

Particularly suitable compositions for oral administration are unit dosage forms such as tablets and capsules. Other fixed unit dosage forms, such as powders presented in sachets, may also be used.

In accordance with conventional pharmaceutical practice the carrier may comprise a diluent, filler, disintegrant, wetting agent, lubricant, colourant, flavourant or other conventional adjuvant.

Typical carriers include, for example, microcrystalline cellulose, starch, sodium starch glycollate, polyvinylpyrrolidone, polyvinylpolypyrrolidone, magnesium stearate or sodium lauryl sulphate.

Most suitably the composition will be formulated in unit dose form. Such unit dose will normally contain an amount of the active ingredient in the range of from 0.1 to 1000 mg, more usually 2-100 mg or 0.1 to 500 mg, and more especially 0.1 to 250 mg.

The present invention further provides a method for treating hyperglycaemia in a human or non-human mammal, which comprises administering an effective, non-toxic, amount of a compound of formula (I), or a pharmaceutically acceptable acid addition salt thereof, or a pharmaceutically acceptable solvate thereof, to a hyperglycaemic human or non-human mammal in need thereof.

The present invention further provides a method for treating obesity or for the treatment and/or prophylaxis of atherosclerosis in a human or non-human mammal, which comprises administering an effective, non-toxic, amount of a compound of formula (I), or a pharmaceutically acceptable acid addition salt thereof, or a pharmaceutically acceptable solvate thereof, to a human or non-human mammal in need thereof.

Conveniently, the active ingredient may be administered as a pharmaceutical composition hereinbefore defined, and this forms a particular aspect of the present invention.

In treating hyperglycaemic or obese humans the compound of formula (I), or a pharmaceutically acceptable salt, ester or amide thereof; or a pharmaceutically acceptable solvate thereof, may be taken in doses, such as those described above, one to six times a day in a manner such that the total daily dose for a 70 kg adult will generally be in the range of from 0.1 to 6000 mg, and more usually about 1 to 1500 mg.

The treatment regimens for atherosclerosis are generally as described for hyperglycaemia.

In treating non-human mammals, especially dogs, the active ingredient may be adminstered by mouth, usually once or twice a day and in an amount in the range of from about 0.025 mg/kg to 25 mg/kg, for example 0.1 mg/kg to 20 mg/kg.

In a further aspect the present invention also provides a method for increasing weight gain and/or improving the feed utilisation efficiency and/or increasing lean body mass and/or decreasing birth mortality rate and increasing post/natal survival rate; of livestock, which method comprises the administration to livestock of an effective non-toxic amount of a compound of formula (I) or a veterinarily acceptable acid addition salt thereof, or a veterinarily acceptable solvate thereof.

Whilst the compounds of formula (I) and the veterinarily acceptable acid addition salts thereof or a veterinarily acceptable solvate thereof, may be administered to any livestock in the abovementioned method, they are particularly suitable for increasing weight gain and/or feed utilisation efficiency and/or lean body mass and/or decreasing birth mortality rate and increasing post-natal survival rate; in poultry, especially turkeys and chickens, cattle, pigs and sheep.

In the preceding method the compounds of formula (I) or veterinarily acceptable acid addition salts thereof will normally be administered orally although non-oral modes of administration, for example injection or implantation, are also envisaged. Suitably the compounds are administered in the feed-stuff or drinking water provided for the livestock. Conveniently these are administered in the feed-stuff at from 10-³ ppm - 500ppm of total daily fed intake, more usually 0.01ppm to 250ppm, suitably less than 100ppm.

The particular formulations used will of course depend upon the mode of administration but will be those used conventionally in the mode of administration chosen. For administration in feed-stuff the drugs are conveniently formulated as a premix in association with a suitable carrier.

Accordingly, the present invention also provides a veterinarily acceptable premix formulation comprising a compound of formula (I), or a veterinarily acceptable acid addition salt thereof; or a veterinarily acceptable solvate thereof, in association with a veterinarily acceptable carrier therefore.

Suitable carriers are inert conventional agents such as powdered starch. Other conventional feed-stuff premix carriers may also be employed.

No unacceptable toxicological effects are expected when compounds of the invention are administered in accordance with the present invention.

The following Examples illustrate the invention but do not limit it in any way.

### Procedure 1

### 4-Formylphenoxymethylethylphosphinate

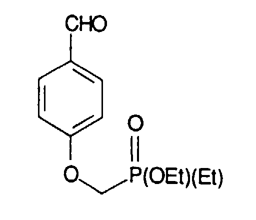

To a suspension of 60% sodium hydride (3.29 g, 82 mMol) in dimethylformamide (DMF) (50 ml) was added dropwise ethylethylphosphinate (10.0 g, 82 mMol) dissolved in DMF (20 ml). After stirring at room temperature for 1.5 h the mixture was cooled to 5°C and treated portionwise with paraformaldehyde (2.56 g, 83 mMol) and stirring continued for 1.5 h. 4-Fluorobenzaldehyde (10.0 g, 82 mMol) was then added and the mixture stirred at room temperature. After 1 h the mixture was poured into saturated ammonium chloride solution (600 ml) and extracted with ethyl acetate (2 x 200 ml). The combined organic layers were washed with water (2 x 200 ml), dried and evaporated to yield the crude product. Chromatography over silica gel eluting with dichloromethane containing 2% methanol gave the title compound as a white, low melting, crystalline solid.
^{**1**}**H NMR δ (CDCl**_{**3**}**)**

9.92 (1H,s); 7.87 (2H,d,J=6.87Hz); 7.08 (2H,d,J=6.87Hz); 4.31 (2H,d,J=7.98Hz); 4.3-4.0 (2H,m); 2.0-1.9 (2H,m); 1.36 (3H,t,J=6.81Hz); 1.25 (3H,dxt,J=7.7,J=15.4Hz).

### Procedure 2

### Ethyl 4-(2-nitropropenyl)phenoxymethylethylphosphinate

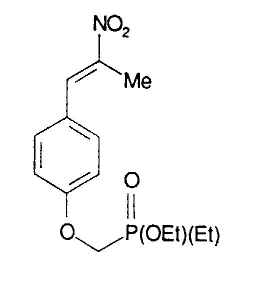

A mixture of ethyl 4-formylphenoxymethylethylphosphinate (12.8 g, 50 mMol) and n-butylamine (5 ml) in benzene (90 ml) was heated at reflux with removal of the water generated. After 1.5 h the mixture was cooled and evaporated to yield a pale yellow oil. Without further purification this was dissolved in a mixture of nitroethane (15 ml) and acetic acid (35 ml) and heated at 100°C for 1.5 h. The resultant dark coloured mixture was cooled and poured into water (400 ml). The organic product was extracted with diethyl ether (3 x 200 ml) and the resultant organic phase washed with water (2 x 100 ml) dried and evaporated to yield the title compound as a dark yellow oil.
^{**1**}**H NMR δ (CDCl**_{**3**}**)**

8.13 (1H,s); 7.69 (2H,d,J=8.3Hz); 7.09 (2H,d,J=8.3Hz); 4.36 (2H,d,J=8.7Hz); 4.3-4.0 (2H,m); 2.45 (3H,s); 2.0-1.9 (2H,m); 1.4-1.0 (6H,m).

### Procedure 3

### Ethyl 4-(2-oxopropyl)phenoxymethylethylphosphinate

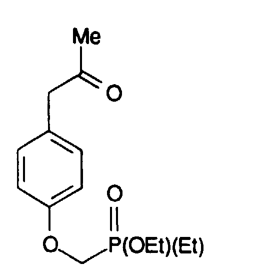

To a mixture of ethyl 4-(2-nitropropenyl)phenoxymethylethylphosphinate (15.0 g, 48 mMol) and iron powder (40 g) suspended in a mixture of methanol (75 ml) and water (25 ml) at reflux was added dropwise acetic acid (40 ml). After 3 h the mixture was cooled, filtered and the solvent evaporated. The residue was partitioned between ethyl acetate (250 ml) water (250 ml) and stirred for 0.5 h, then filtered and the organic layer separated. The aqueous phase was extracted with ethyl acetate (150 ml) and the combined organic layers washed with water, dried and evaporated to yield the crude product Chromatography over silica gel eluting with dichloromethane containing 3% methanol gave the title compound as a pale yellow oil.
^{**1**}**H NMR δ (CDCl**_{**3**}**)**

7.13 (2H,dxd,J=1.9 and 8.8Hz); 6.92 (2H,dxd,J=2.2 and 8.79Hz); 4.22 (2H,d,J=7.15Hz); 4.2-4.0 (2H,m); 3.65 (2H,s); 2.16 (3H,s); 1.95 (2H,dxt,J=5.49 and 18.37Hz); 1.34 (3H,t,J=7.15Hz); 1.23 (3H,dxt,J=7.42 and 18.97Hz).

### Procedure 4

### (RR) Ethyl 4-[2-(1-methyl-1-phenyl)aminopropyl]phenoxymethylethyl phosphinate hydrochloride

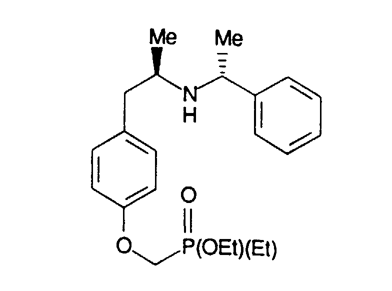

A mixture of ethyl 4-(2-oxopropyl)phenoxymethylethyl phosphinate (4 g, 14.1 mMol), (R) α-methylbenzylamine (1.7 g, 14.1 mMol) and platinum IV oxide (30 mg) in methanol (50 ml) was hydrogenated at atmospheric pressure. After 24 h the reaction mixture was filtered and the solvent evaporated to yield a pale yellow oil. Chromatography over silica gel eluting with dichloromethane containing methanol 3% gave an oil. This product was dissolved in ethyl acetate (25 ml) and dry hydrogen chloride was passed through the solution for 10 minutes. The solvent was evaporated to yield the title compound as a white foam. [α]^{D} ₂₅ MeOH [1%]= +28.0°
^{**1**}**H NMR δ (D**^{**6**}**-DMSO + D**_{**2**}**O)**

7.36-7.14 (5H,m); 7.02 (2H,d,J=8.8Hz); 6.84 (2H,d,J=8.8Hz); 4.20 (2H,d,J=9.08Hz); 4.23-4.05 (2H,m); 3.92 (1H,q,J=6.32Hz); 2.84-2.68 (2H,m); 2.45 (1H,dxd,J=6.88 and 12.65Hz); 1.96 (2H,dxt,J=5.5 and 18.5Hz); 1.33 (3H,t,J=7.15Hz); 1.26 (3H,t,J=7.7Hz); 1.20 (3H,d,J=7.7Hz); 0.89 (3H,d,J=6.05Hz).

### Procedure 5

### (R) Ethyl 4-(2-aminopropyl)phenoxymethylethylphosphinate

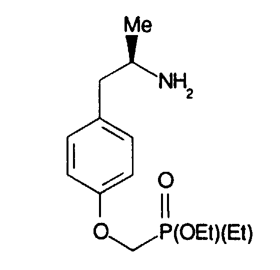

A mixture of (RR) ethyl 4-[2-(1-methyl-1-phenyl)aminopropyl]phenoxymethylethylphosphinate (1.5 g, 3.63 mMol), ammonium formate (2 g) and 10% palladium on charcoal (50 mg) in methanol (30 ml) was heated at reflux. After 3 h the reaction mixture was cooled and filtered and the filtrate evaporated to yield a pale yellow oil. The crude product was partitioned between dichloromethane (30 ml) and water (30 ml). The organic layer was then dried and evaporated to yield a viscous yellow oil. [α]^{D} ₂₅ MeOH [1%]= .6.0°
^{**1**}**H NMR δ (CDCl**_{**3**}**)**

7.12 (2H,d,J=8.8Hz); 6.89 (2H,d,J=8.8Hz); 4.2-4.2 (4H,m); 3.2-3.1 (1H,m); 2.66 (1H,dxd,J=5.22 and 13.33Hz); 2.46 (1H,dxd,J=13.46 and 7.97); 1.92-1.87 (2H,m); 1.50-1.25 (2H,m,exchanges with D₂O); 1.35 (3H,t,J=7.14Hz); 1.23 (3H,dxt,J=7.7 and 18.7Hz); 1.10 (3H,d,J=6.33Hz).

### Procedure 6

### (R)-1-(3-Chlorophenyl)-1-hydroxy-2-iodoethane

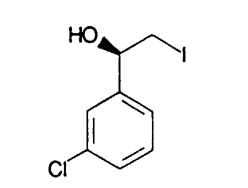

A degassed mixture of (R)-1-(3-chlorophenyl)-2-chloro-1-hydroxyethane (2.9 g, 15.1 mMol) and sodium iodide (10 g, 66 mMol) in acetone (30 ml) was heated at reflux under an atmosphere of argon. After 72 h the mixture was cooled and diethyl ether (100 ml) added. The resultant solution was washed with water (100 ml), 10% sodium metabisulfite solution (100 ml), brine and finally dried and evaporated to yield the title compound as a brown oil. [α]^{D} ₂₅ cyclohexane [1%]= -21°
^{**1**}**H NMR δ (CDCl**_{**3**}**)**

7.39 (1H,s); 7.30-7.20 (3H,m); 4.80 (1H,dxd,J=3.58 and 8.79Hz); 3.48 (1H,dxd,J=3.57 and 10.17Hz); 3.36 (1H,dxd,J=10.17 and 8.79Hz); 2.6-2.5 (1H,br s,exchanges with D₂O).

### Procedure 7

### (R)-1-(3-Chlorophenyl)-2-iodo-1-triethylsilyloxyethane

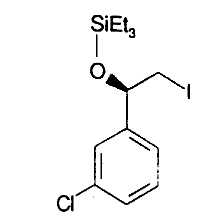

A mixture of imidazole (2.5 g, 37 mMol) (R)-1-(3-chlorophenyl)-1-hydroxy-2-iodoethane (3.5 g, 12.7 mMol) and 4-dimethylaminopyridine (70 mg) in DMF under an atmosphere of argon was treated dropwise with triethylsilyltrifluoromethanesulfonate (2.6 ml, 15.2 mMol). After 0.5 h the resultant mixture was diluted with 3:1 hexane/ethyl acetate (50 ml), washed with water (20 ml), saturated copper sulfate solution (20 ml) and brine (20 ml). Finally the organic layer was dried and evaporated to yield the title compound as a pale brown oil. [α]^{D} ₂₅ cyclohexane [1%]=-25.0°
^{**1**}**H NMR δ (CDCl**_{**3**}**)**

7.34 (1H,s); 7.30-7.20 (3H,m); 4.77 (1H,t,J=5.78Hz); 3.30 (2H,d,J=5.85Hz); 1.00-0.80 (9H,m); 0.6-0.4 (6H,m).

### Procedure 8

### (RR)-Diethyl 4-[2-[2-(3-chlorophenyl)-2-triethylsilyloxyethylamino]propyl]phenoxymethylethylphosphinate

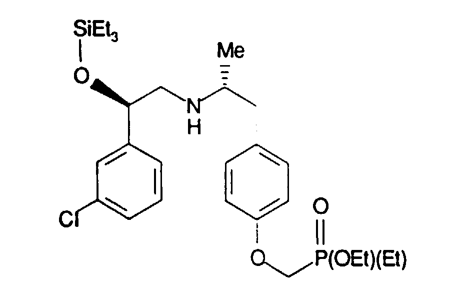

A mixture of (R)-ethyl 4-(2-aminopropyl)phenoxymethylethylphosphinate (0.76 g, 2.66 mMol) triethylamine (0.75 ml, 5.32 mMol) and (R)-1-(3-chlorophenyl)-2-iodo-1-triethylsilyloxyethane (0.75 g, 1.89 mMol) in THF (4 ml) was heated in a sealed tube at 100°C in an argon atmosphere. After 24 h a biphasic mixture had formed, this was partitioned between ethyl acetate (25 ml) and water (25 ml). The organic layer was separated and the aqueous phase extracted with ethyl acetate (25 ml). The combined organic phases were combined, dried and evaporated to yield a straw coloured oil. Chromatography over silica gel eluting with dichloromethane containing methanol 2% gave the title compound as a yellow gum.
^{**1**}**H NMR δ (CDCl**_{**3**}**)**

7.30-7.15 (4H,m); 7.07-7.03 (2H,m); 6.90-6.75 (2H,m); 4.75-4.70 (1H,m); 4.30-4.05 (4H,m); 2.9-2.7 (1H,m); 2.60-2.55 (2H,m); 2.55-2.45 (1H,m); 2.01-1.80 (2H,m); 1.75-1.53 (1H,br s,exchanges with D₂O); 1.40-1.20 (6H,m); 1.02 (3H,t,J=12.1Hz); 0.90-0.75 (9H,m); 0.60-0.30 (6H,m).

### Procedure 9

### n-Butylphenylphosphite

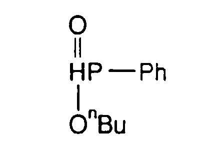

To n-butanol (25 ml) at 5°C was added dropwise with stirring dichlorophenylphosphine (20g, 0.112 Mol). The resultant mixture was then stirred at room temperature under a vacuum of ca. 25 mm of mercury. After 3 hours the vacuum was increased to 15 mm of mercury and the temperature raised to 50°C for 1 hour.

The residue, a colourless, mobile oil, afforded the pure title compound.

¹H NMR δ(CDCl₃)
7.8 - 7.7 (2H,m); 7.6-7.5 (3H,m); 7.58 (1H, d, J = 563 Hz); 4.2 - 4.0 (2H,m);
1.80 - 1.60 (2H,m); 1.5 - 1.3 (2H,m); 0.93 (3H, t, J = 7.15 Hz).

### Procedure 10

### n-Butyl 4-formylphenoxymethylphenylphosphinate

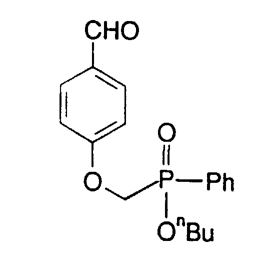

The title compound was obtained from n-butylphenylphosphite (16.2 g, 81.7 mMol), 60% sodium hydride (3.27g, 81.7 mMol), paraformaldehyde (2.45g, 81.7 mMol) and 4-fluorobenzaldehyde (8.76 ml, 81.7 mMol) in an analogous manner to that described in Procedure 1. The crude compound was purified by chromatography over silica gel eluting with ethyl acetate containing 2% methanol to yield a colourless oil.

¹H NMR δ(CDCl₃)
9.88 (1H, s); 8.03 - 7.80 (4H,m); 7.62 - 7.48 (3H, m);
6.97 (2H, d, J = 8.53Hz); 4.59 - 4.38 (2H, m); 4.20 - 4.03 (2H, m);
1.73 - 1.65 (2H, m); 1.46 - 1.37 (2H, m): 0.91 (3H, t, J = 7.15 Hz).

### Procedure 11

### n-Butyl 4-(2-nitropropenyl)phenoxymethylphenyl phosphinate

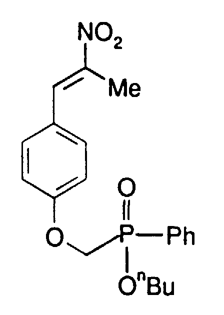

The title compound was obtained in a two-step sequence: initially n-butyl 4-formyl phenoxymethylphenylphosphinate (13.0 g, 13.6 mMol) and n-butylamine (30 ml) in toluene (150 ml) gave the corresponding imine. Without further purification this intermediate was treated with nitroethane (20 ml) and acetic acid (40 ml) in an analogous manner to that described in Procedure 2.

¹H NMR δ(CDCl₃)
8.10-7.75 (3H, m); 7.65 - 7.28 (3H, m); 7.39 (2H, d, J = 9Hz);
6.98 (2H, d, J = 9Hz); 4.51 - 4.37 (2H, m); 4.30 - 3.95 (2H, m);
2.40 (2H, s); 2.16 (1H, s); 1.85 - 1.20 (4H, m); 0.88 (3H, t, J = 6.3Hz).

### Procedure 12

### n-Butyl 4-(2-oxopropyl)phenoxymethylphenyl phosphinate

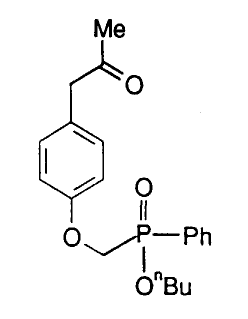

The title compound was obtained from n-butyl 4-(2-nitropropenyl) phenoxymethylphenyl phosphinate (6.66g, 18.6 mMol), iron powder (25g) and acetic acid (25 ml) in a mixture of water (30 ml) and methanol (75 ml). The crude compound was chromatographed over silica gel eluting with a 1:1 mixture of ethyl acetate and hexane to yield a pale yellow oil.

¹H NMR δ(CDCl₃)
7.97 - 7.89 (2H, m); 7.59 - 7.49 (3H, m); 7.09 (2H, d, J = 8.79 Hz);
6.87 (2H, d, J = 8.80 Hz); 4.51 - 4.28 (2H, m); 4.14 - 4.04 (2H, m);
3.62 (2H, s); 2.12 (3H, s); 1.73 - 1.67 (2H, m); 1.46 - 1.37 (2H, m);
0.91 (3H, t, J = 7.42 Hz).

### Procedure 13

### n-Butyl 4-[2-(2-dioxalano)propyl]phenoxymethylphenyl phosphinate

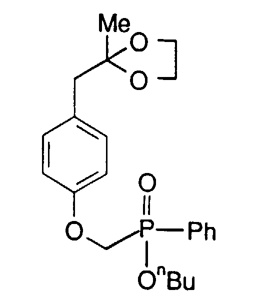

A mixture of n-butyl 4-(2-oxopropyl)phenoxymethylphenyl phosphinate (2.0g, 5.55mMol), 1,2-ethanediol (10 ml) and 4-toluenesulphonic acid (50 mg) in toluene (100 ml) was heated under reflux for 24 hours with concomitant removal of water. The solution was washed with water (50 ml), dried and evaporated to yield the title compound as a light yellow oil.

¹H NMR δ(CDCl₃)
7.96-7.89 (2H, m), 7.53-7.49 (3H, m); 7.15 (2H, d, J = 8.8 Hz);
6.81 (2H, d, J = 8.8 Hz); 4.44 - 4.31 (2H, m); 4.14 - 4.04 (2H, m);
3.89 - 3.86 (2H, m); 3.74 - 3.71 (2H, m); 2.84 (2H, s);
1.73 - 1.67 (2H, m); 1.43 - 1.40 (2H, m); 1.26 (3H, s);
0.91 (3H, t, J = 7.42 Hz).

### Procedure 14

### Lithium 4-[2-(2-dioxalano)propyl]phenoxymethylphenyl phosphinate

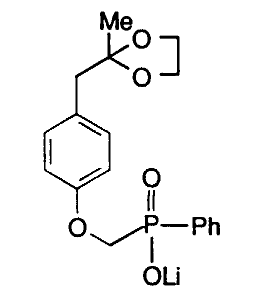

A mixture of n-butyl 4-[2-(2-dioxalano)propyl]phenoxymethylphenylphosphinate (2.02 g, 5 mMol) and 1M lithium hydroxide (10 ml) in dioxane (10 ml) was stirred for 24 hours at room temperature. The solvent was evaporated to yield the title compound as a pale yellow solid, contaminated with lithium hydroxide.

¹H NMR δ(CD₃OD/D₂O)
8.1 - 7.5 (2H, m); 7.4 - 7.2 (3H, m); 7.12 (2H, d, J = 8.4 Hz);
6.84 (2H, d, J = 8.4 Hz); 4.19 (2H, d, J = 7.8 Hz);
3.7 - 3.4 (4H, m); 2.70 (2H, s), 1.26 (3H, s).

### Procedure 15

### 4-(2-Oxopropyl)phenoxymethylphenylphosphinic acid

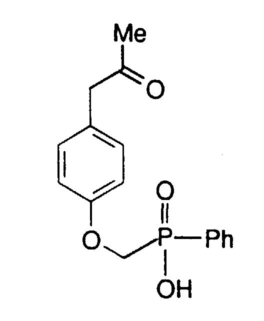

A mixture of lithium 4-[2-(2-dioxalano)propyl]phenoxymethylphenylphosphinate (2.07g, 5.84 mMol) and 1M hydrochloric acid (10 ml) in acetone (15 ml) was stirred for 20 hours at room temperature.

The solvent was then evaporated and the residue partitioned between water (50 ml) and a mixture of 3 parts ethyl acetate and 1 part isopropanol (100 ml). The layers were separated and the organic layer was washed with water (50 ml) and brine (50 ml). Drying and evaporation yielded the title compound as a yellow non-crystalline solid.

¹H NMR δ(D⁶ DMSO + D₂O)
7.96 - 7.89 (2H, m), 7.63 - 7.56 (3H, m); 7.12 (2H, d, J = 8.52 Hz);
6.91 (2H, d, J = 8.52 Hz); 4.34 (2H, d, J = 8.52 Hz); 3.73 (2H, s); 2.15 (3H, s).

### Example 1

### (RR)-Lithium 4-[2-[2-(3-chlorophenyl)-2-hydroxyethylamino]propyl]phenoxymethylethylphosphinate

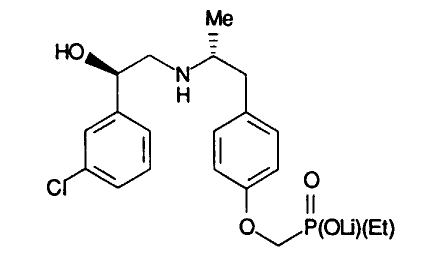

A solution of (RR)-diethyl 4-[2-[2-(3-chlorophenyl)-2-triethylsilyloxyethylamino]propyl]phenoxymethylethylphosphinate (240 mg, 0.43 mMol) in acetone (5 ml) was treated with 6 M hydrochloric acid (1 ml) under an atmosphere of argon. After 3 h at room temperature the acetone was evaporated and the residue was treated with a slight excess of sodium bicarbonate. The organic material was extracted with dichloromethane (2x25 ml) and the combined organic phases dried and evaporated to yield a gum.

Without further purification the crude compound (140 mg, 0.32 mMol) was dissolved in dioxane (2 ml) and treated with 1 M lithium hydroxide (2 ml). After stirring overnight at room temperature the dioxan was evaporated and water (4 ml) added, the resultant crystalline solid, mp 260°C decomp.
^{**1**}**H NMR δ (D**^{**6**} **DMSO + D**_{**2**}**O)**

7.34-7.18 (4H,m): 7.08 (1H,d,J=8.61 Hz); 7.03 (1H,d,J=8.59Hz); 6.91 (1H,d,J=8.63Hz); 6.87 (1H,d,J=8.64Hz); 4.66 (1H,m); 3.95 (1H,d,J=8.42Hz); 3.94 (1H,d,J=8.42Hz); 2.95-2.82 (2H,m); 2.70-2.50 (3H,m); 1.13 (6H,dxdxt,J=1.87,7.65 and 17.03Hz); 1.06 (3H,dxd,J=1.55 and 6.28).

### Example 2

### (RR RS) Ethyl 4-[2-[2-(3,4-dihydroxyphenyl)-2-hydroxyethylamino]propyl]phenoxymethylethylphosphinate

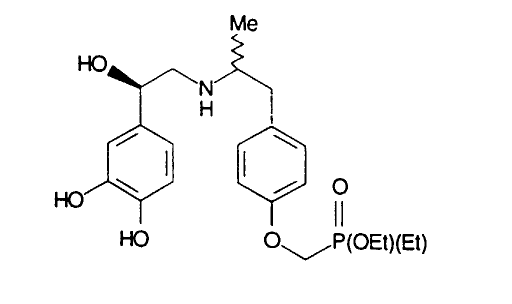

A mixture of (R)-noradrenaline (0.5 g, 2.95 mMol) and ethyl 4-(2-oxopropyl)phenoxymethylethylphosphinate (0.84 g, 2.95 mMol) was stirred for 1 h at room temperature. 10% Palladium on charcoal (0.3 g) was then added and the resultant mixture hydrogenated at 50 psi and 50°C. After 16 h the reaction was cooled, filtered and the solvent evaporated to yield a foam. Treatment of this foam with diethyl ether gave the title compound as a free flowing white powder, mp 154-6°C.

^{**1**}**H NMR δ (D**^{**6**} **DMSO + D**_{**2**}**O)**

7.09 (1H,d,J=8.54Hz); 7.05 (1H,d,J=8.59Hz); 6.92 (2H,dxd,J=8.72 and 10.02Hz); 6.71 (1H,s); 6.63 (1H,dxd,J=1.78 and 7.99Hz); 6.53 (1H,m); 4.44-4.35 (1H,m); 4.32-4.24 (2H,m); 4.09-3.98 (2H,m); 2.79 (1H,q,J=6.75Hz); 2.67-2.60 (3H,m); 2.47-2.39 (2H,m); 1.87-1.78 (2H,m); 1.23 (3H,t,J=7.03Hz); 1.07 (3H,dxt,J=7.69 and 18.27Hz); 0.90 (3H,d,J=6.14Hz).

### Example 3

### (RR RS) 4-[2-[2-(3,4-Dihydroxyphenyl)-2-hydroxyethylamino]propyl]phenoxymethyl phenylphosphinic acid.

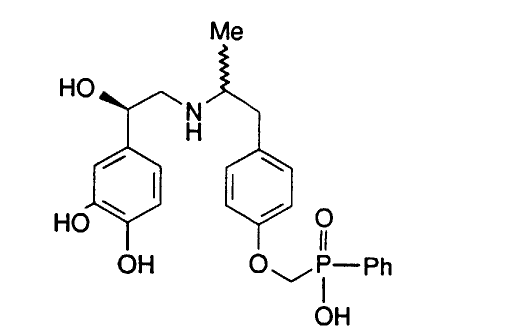

The title compound was obtained from 4-(2-oxopropyl)phenoxymethylphenyl phosphinic acid (440 mg, 1.47 mMol), (R)-noradrenaline (250 mg, 1.47 mMol) and 10% palladium charcoal (300 mg) in methanol (50 ml) in an analogous manner to that described in Example 2. The crude product was purified by chromatography over C₁₈-reverse phase silica eluting with water containing methanol (30%) to yield a white solid.

¹H NMR δ(CD₃OD)
7.90-7.84(2H,m); 7.49-7.43(3H,m); 7.07(1H,d, J = 8.44Hz);
7.03 (1H,d, J = 8.40Hz); 6.88 - 6.77 (4H,m); 6.66 (1H,d, J = 8.28 Hz);
4.53 - 4.49 (1H,m); 4.12 (2H, d, J = 8.34 Hz); 3.13 - 3.01 (1H,m);
2.92 - 2.82 (2H,m); 2.72 - 2.61 (2H,m); 1.20 (1.5H, d, J = 5.61Hz);
1.18(1.5H, d, J = 5.96Hz).

### Pharmacological Data

The activity of the present compounds may be tested by use of the following procedures:

### Lipolysis

Rat white adipocytes are prepared by collagenase digestion as described by Rodbell (1964) with the modification of Honnor et al. (1985), in that adenosine (200nM) are included in the preparation to inhibit basal lipolysis.

Lipolysis stimulated by β-agonists is measured over 30 minutes in medium where the adenosine concentration is controlled; the medium included (-)-N⁶-(2-phenylisopropyl)adenosine (100nM) + deaminase as decribed by Honnor et al. (1985). The incubation is stopped by addition of trichloracetic acid (0.2ml of 10% w/v) to 1ml of cells. Glycerol is measured by the fluorimetric method of Boobis and Maugham (1983).

### References

1. Rodbell M. (1964) **J. Biol. Chem.** 239, 375-380
2. Honnor R.C., Dhellon G.S. and Londos C. (1985) **J. Biol. Chem.** 260, 15122-15129
3. Boobis L.H. and Maugham R.J. (1983) **Clinica Chemica Acta** 132, 173-179.

### Agonist Activity at Rat β₁ and β₂ Adrenoceptors In Vitro

### β₁-Adrenoceptor Agonism

Female Sprague-Dawley rats (150-250g) are killed by a blow to the head and exsanguinated. Spontaneously beating right atria are removed by the method of Broadley and Lumley (1977) and mounted on a glass tissue holder. Each tissue is placed in 30 ml organ baths at 37°C containing Kreb's-Henseleit solution. Each atrium is attached to an isometric transducer by cotton and placed under an initial resting tension of 1g. Rate recordings from the spontaneous beating atria are obtained from the tension signal using a Lectromed Type 4522 ratemeter. All traces are recorded on a Lectromed M4 chart recorder. β-adrenoceptor agonists are then added to the Krebs medium in a cumulative fashion and the results expressed as a percentage increase in atrial rate.

### β₂-Adrenoceptor Agonism

Rat uterine horns are removed and bisected longitudinally. Each tissue is tied to a glass tissue holder and placed in Krebs-Henseleit solution in a 30 ml organ bath as before. Tissues are placed under a resting tension of 1g and allowed to equilibrate. Each uterine strip is pre-contracted by the addition of 40mM K⁺ to the bath to produced a steady tonic contraction. β-agonists are then added to the bath in a cumulative manner and results expressed as percentage inhibition of contraction.

Agonist EC₅₀ (atria) and IC₅₀ (uteri) are calculated as the concentration of agonist producing 50% of their maximum increase in atrial rate or uterine relaxation. Relative intrinsic activity expressed as the maximal responses to test agonists relative to isoprenaline (=1.0) in both atria and uteri.

### References

K.J. Broadley & P. Lumley (1977) **Br. J. Pharmacol. 59.51**

### Agonist Activity at Human β₁, β₂, and β₃-Adrenoceptors.

Subclones of CHO cells were stably transfected with each of the human β₁, β₂ and β₃-adrenoceptors¹. Cells were then disrupted by immersion in ice-cold lysis buffer (10 mM TRIS, 2mM EDTA , pH 7.4) containing protease inhibitors leupeptin and benzamidine (5 µg / ml) and soyabean trypsin inhibitor (10 µg / ml). Membranes were prepared by the method of Bouvier et. al.² and stored in 1 ml aliquots in liquid N₂ for future use.

### β₃-Adrenoceptor- Mediated Adenylyl Cyclase Activity

Adenylyl cyclase activity was assayed by the method of Kirkham et. al.³ by the addition of 40 µl (70 -80 µg protein) to the incubation medium of the above CHO cell plasma membranes transfected with the human β₃-adrenoceptor . cAMP produced over 20 minutes was separated from ATP by the method of Salomon et al.⁴. Agonist EC₅₀ values and intrinsic activities were expressed as the concentration of agonist producing 50 % activation of adenylyl cyclase and the maximum response produced by each agonist relative to that produced by (-) isoprenaline respectively.

| | EC₅₀(µM) |
|---|---|
| Example 2 | 0.46 |
| Example 3 | 0.54 |

### References

1. T. Frielle et. al., Proc. Natl. Acad. Sci., 1987, **84**, 7920; B. Kobilka, Proc. Natl. Acad. Sci., 1987, **84**, 46; S. Liggett and D. Schwinn, DNA Sequence, 1991, **2**, 61.
2. M. Bouvier et. al., Mol. Pharmacol., 1987, **33**, 133.
3. D. Kirkham et. al., Biochem. J., 1992, **284**, 301.
4. Y. Salomon et al., Anal. Biochem., 1974, **58**, 541.

## Claims

1. A compound of formula (I): 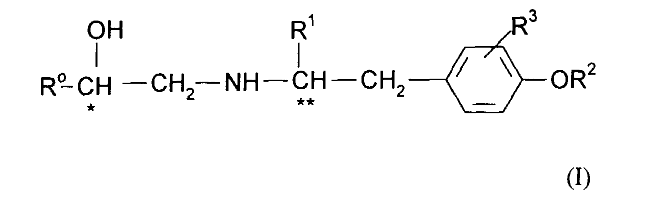 or a pharmaceutically acceptable salt thereof, or a pharmaceutically acceptable solvate thereof, wherein,
R^{o} represents a substituted or unsubstituted aryl group;
R¹ represents hydrogen or an alkyl group;
R² represents a moiety of formula (a): 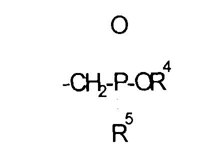 wherein R⁴ and R⁵ each independently represent hydrogen, alkyl, phenyl, hydroxyalkyl or cycloalkyl and R³ represents hydrogen, halogen, alkyl or alkoxy.

2. A compound according to claim 1, wherein R^{o} is a substituted phenyl group.

3. A compound according to claim 1 or claim 2, wherein R^{o} is 3-chlorophenyl or 3,4-dihydroxyphenyl.

4. A compound according to any one of claims 1 to 3, wherein R⁵ represents alkyl.

5. A compound according to any one of claims I to 4, wherein R⁴ is hydrogen.

6. A compound according to any one of claims 1 to 4, wherein R⁴ is an alkyl group.

7. A compound according to any one of claims 1 to 6, wherein R³ is hydrogen.

8. A compound according to any one of claims 1 to 7 in the form of an RR enantiomer.

9. A compound according to claim 1, selected from the list consisting of:
(RR)-lithium 4-[2-[2-(3-chlorophenyl)-2-hydroxyethylamino]propyl]phenoxymethylethylphosphinate;
RR RS) ethyl 4-[2-[2-(3,4-dihydroxyphenyl)-2-hydroxyethylamino]propyl]phenoxymethylethylphosphinate; and
(RR RS) 4-[2-[2-(3,4-dihydroxyphenyl)-2-hydroxyethylamino]propyl]phenoxymethyl phenylphosphinic acid;or a pharmaceutically acceptable salt thereof, or a pharmaceutically acceptable solvate thereof

10. A process for the preparation of a compound of formula (I), or a pharmaceutically acceptable acid addition salt or a pharmaceutically acceptable solvate thereof, which process comprises:
(a) reducing a compound of formula (II): 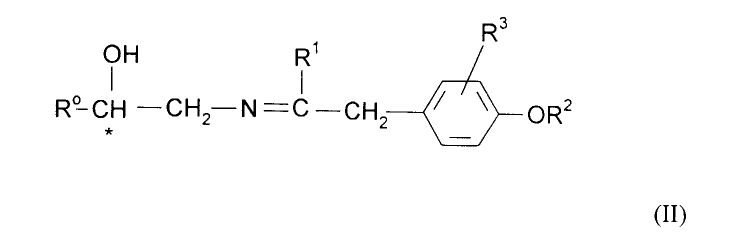 wherein R^{o}, R¹, R² and R³ are as defined in relation to formula (I) in claim 1; or
(b) deprotecting a compound of formula (IX): 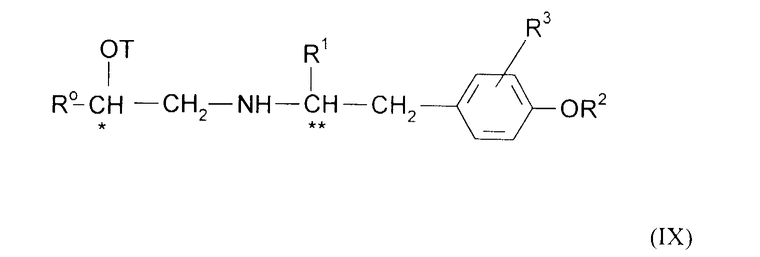 wherein R^{o}, R¹, R² and R³ are as defined in relation to formula (I) and T represents an hydroxy protecting group;
and thereafter, if necessary, carrying out one or more of the following optional steps:
(i) converting one compound of formula (I) to another compound of formula (I); and
(ii) preparing a pharmaceutically acceptable acid addition salt of a compound of formula (I) or a pharmaceutically acceptable solvate thereof.

11. A pharmaceutical composition comprising a compound of formula (I), or a pharmaceutically acceptable salt thereof; or a pharmaceutically acceptable solvate thereof, and a pharmaceutically acceptable carrier therefor.

12. A compound of formula (I), or a pharmaceutically acceptable salt thereof; or a pharmaceutically acceptable solvate thereof, for use as an active therapeutic substance.

13. A compound of formula (I), or a pharmaceutically acceptable salt thereof; or a pharmaceutically acceptable solvate thereof, for use in the treatment of hyperglycaemia, obesity, atherosclerosis, hyperinsulinaemia, gastrointestinal disorders or the treatment of gastrointestinal ulcerations.

14. The use of a compound of formula (I), or a pharmaceutically acceptable salt thereof; or a pharmaceutically acceptable solvate thereof, for the manufacture of a medicament for the treatment of hyperglycaemia, obesity, atherosclerosis, hyperinsulinaemia, gastrointestinal disorders or the treatment of gastrointestinal ulcerations.

15. A method for increasing weight gain and/or improving the feed utilisation efficiency and/or increasing lean body mass of livestock, which method comprises the administration to livestock of an effective non-toxic amount of a compound of formula (I) or a veterinarily acceptable salt thereof; or a veterinarily acceptable solvate thereof.

16. A veterinarily acceptable premix formulation comprising a compound of formula (I), or a veterinarily acceptable salt thereof; or a veterinarily acceptable solvate thereof, in association with a veterinarily acceptable carrier therefore.

17. A compound, being an intermediate:
(a) of formula (II): 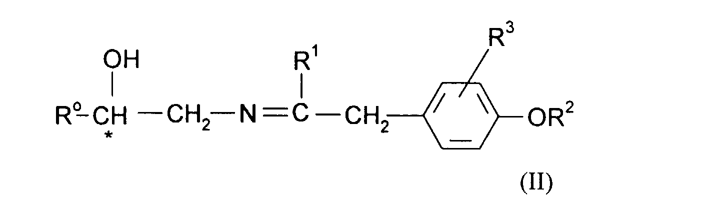 wherein R^{o}, R¹, R² and R³ are as defined in relation to formula (I) in claim 1;
(b) of formula (IX): 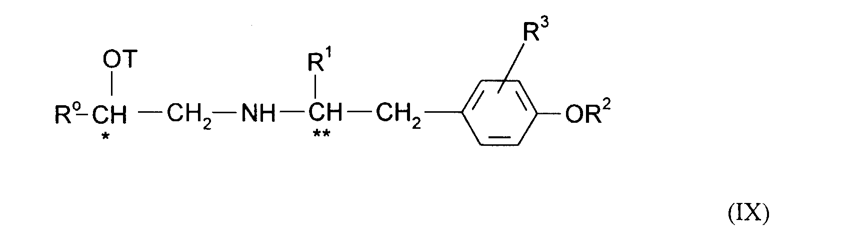 wherein R^{o}, R¹, R² and R³ are as defined in relation to formula (I) and T represents an hydroxy protecting group

## Patentansprüche

1. Verbindung der Formel (I) 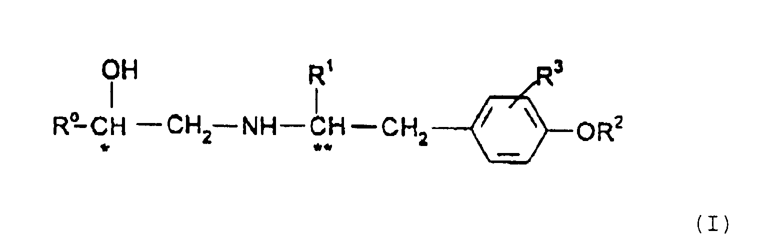 oder ein pharmazeutisch verträgliches Salz davon, oder ein pharmazeutisch verträgliches Solvat davon, in der
R⁰ eine substituierte oder unsubstituierte Arylgruppe bedeutet;
R¹ ein Wasserstoffatom oder eine Alkylgruppe bedeutet; R² einen Rest der Formel (a) bedeutet: 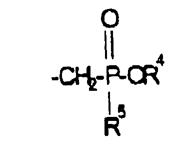 in der R⁴ und R⁵ jeweils unabhängig voneinander ein Wasserstoffatom, eine Alkyl-, Phenyl-, Hydroxyalkyl- oder Cycloalkylgruppe bedeuten, und R³ ein Wasserstoffatom, ein Halogenatom, eine Alkylgruppe oder eine Alkoxygruppe bedeutet.

2. Verbindung nach Anspruch 1, in der R⁰ eine substituierte Phenylgruppe ist.

3. Verbindung nach Anspruch 1 oder 2, in der R⁰ 3-Chlorphenyl oder 3,4-Dihydroxyphenyl ist.

4. Verbindung nach einem der Ansprüche 1 bis 3, in der R⁵ eine Alkylgruppe ist.

5. Verbindung nach einem der Ansprüche 1 bis 4, in der R⁴ ein Wasserstoffatom ist.

6. Verbindung nach einem der Ansprüche 1 bis 4, in der R⁴ eine Alkylgruppe ist.

7. Verbindung nach einem der Ansprüche 1 bis 6, in der R³ ein Wasserstoffatom ist.

8. Verbindung nach einem der Ansprüche 1 bis 7, in Form eines RR-Enantiomers.

9. Verbindung nach Anspruch 1, ausgewählt aus
(RR)-Lithium-4-[2-[2-(3-chlorphenyl)-2-hydroxyethylamino]propyl]phenoxymethylethylphosphinat;
(RR RS)-Ethyl-4-[2-[2-(3,4-dihydroxyphenyl)-2-hydroxyethylamino]propyl]phenoxymethylethylphosphinat; und
(RR RS)-4-[2-[2-(3,4-Dihydroxyphenyl)-2-hydroxyethylamino]propyl]phenoxymethylphenylphosphinsäure; oder ein pharmazeutisch verträgliches Salz davon oder ein pharmazeutisch verträgliches Solvat davon.

10. Verfahren zur Herstellung einer Verbindung der Formel (I) oder eines pharmazeutisch verträglichen Säureadditionssalzes oder eines pharmazeutisch verträglichen Solvats davon, umfassend
(a) Reduzieren einer Verbindung der Formel (II) 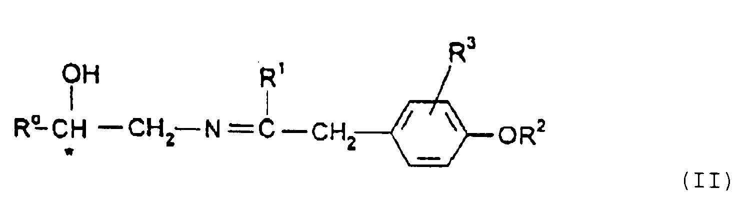 in der R⁰, R¹, R² und R³ wie in Anspruch 1 mit Bezug auf Formel (I) definiert sind; oder
(b) Abspalten einer Schutzgruppe von einer Verbindung der Formel (IX) 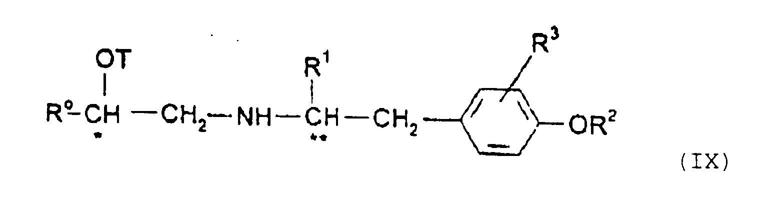 in der R⁰, R¹, R² und R³ wie mit Bezug auf Formel (I) definiert sind und T eine Hydroxyschutzgruppe bedeutet;
und anschließend, wenn nötig, Durchführen eines oder mehrerer der folgenden möglichen Schritte:
(i) Überführen einer Verbindung der Formel (I) in eine andere Verbindung der Formel (I); und
(ii) Herstellen eines pharmazeutisch verträglichen Säureadditionssalzes einer Verbindung der Formel (I) oder eines pharmazeutisch verträglichen Solvats davon.

11. Arzneimittel umfassend eine Verbindung der Formel (I) oder ein pharmazeutisch verträgliches Salz davon oder ein pharmazeutisch verträgliches Solvat davon, sowie einen pharmazeutisch verträglichen Träger.

12. Verbindung der Formel (I) oder ein pharmazeutisch verträgliches Salz davon oder ein pharmazeutisch verträgliches Solvat davon zur Verwendung als therapeutischer Wirkstoff.

13. Verbindung der Formel (I) oder ein pharmazeutisch verträgliches Salz davon oder ein pharmazeutisch verträgliches Solvat davon zur Verwendung in der Behandlung von Hyperglykämie, Fettsucht, Atherosklerose, Hyperinsulinämie, Magen-Darm-Störungen oder der Behandlung von Geschwürsbildungen im Magen-Darm-Bereich.

14. Verwendung einer Verbindung der Formel (I) oder eines pharmazeutisch verträglichen Salzes davon oder eines pharmazeutisch verträglichen Solvats davon zur Herstellung eines Medikaments zur Behandlung von Hyperglykämie, Fettsucht, Atherosklerose, Hyperinsulinämie, Magen-Darm-Störungen oder der Behandlung von Geschwürsbildungen im Magen-Darm-Bereich.

15. Verfahren zum Steigern der Gewichtszunahme und/oder der Futterverwertung und/oder der fettarmen Körpermasse von Nutztieren, umfassend die Verabreichung einer wirksamen nichttoxischen Menge einer Verbindung der Formel (I) oder eines tiermedizinisch verträglichen Salzes oder eines tiermedizinisch verträglichen Solvats davon.

16. Tiermedizinisch verträgliche Vorformulierung umfassend eine Verbindung der Formel (I) oder ein tiermedizinisch verträgliches Salz oder ein tiermedizinisch verträgliches Solvat davon, in Verbindung mit einem tiermedizinisch verträglichen Träger.

17. Verbindung als Zwischenverbindung von
(a) Formel (II) 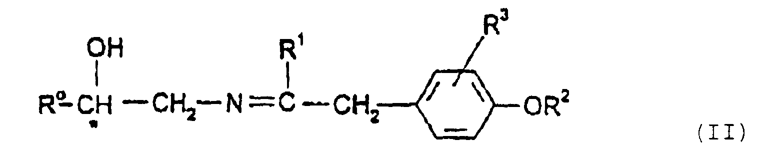 in der R⁰, R¹, R² und R³ wie in Anspruch 1 mit Bezug auf Formel (I) definiert sind;
(b) Formel (IX) 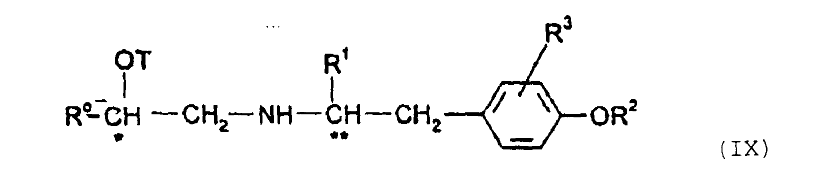 in der R⁰, R¹, R² und R³ wie mit Bezug auf Formel (I) definiert sind und T eine Hydroxyschutzgruppe bedeutet.

## Revendications

1. Composé de formule (I) : 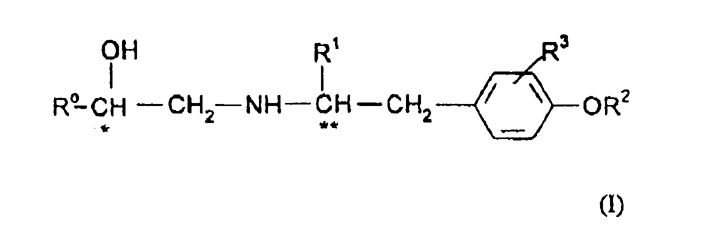 ou un de ses sels pharmaceutiquement acceptables, ou bien un de ses produits de solvatation pharmaceutiquement acceptables, formule dans laquelle
R⁰ représente un groupe aryle substitué ou non substitué ;
R¹ représente l'hydrogène ou un groupe alkyle ;
R² représente un groupe de formule (a) : 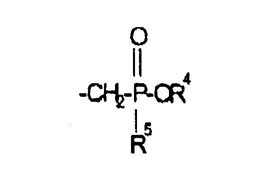 dans laquelle R⁴ et R⁵ représentent chacun indépendamment l'hydrogène, un groupe alkyle, phényle, hydroxyalkyle ou cycloalkyle et R³ représente l'hydrogène, un halogène, un groupe alkyle ou alkoxy.

2. Composé suivant la revendication 1, dans lequel R⁰ représente un groupe phényle substitué.

3. Composé suivant la revendication 1 ou la revendication 2, dans lequel R⁰ représente un groupe 3-chlorophényle ou 3,4-dihydroxyphényle.

4. Composé suivant l'une quelconque des revendications 1 à 3, dans lequel R⁵ représente un groupe alkyle.

5. Composé suivant l'une quelconque des revendications 1 à 4, dans lequel R⁴ représente l'hydrogène.

6. Composé suivant l'une quelconque des revendications 1 à 4, dans lequel R⁴ représente un groupe alkyle.

7. Composé suivant l'une quelconque des revendications 1 à 6, dans lequel R³ représente l'hydrogène.

8. Composé suivant l'une quelconque des revendications 1 à 7, sous forme d'un énantiomère RR.

9. Composé suivant la revendication 1, choisi dans la liste des composés suivants :
4-[2-[2-(3-chlorophényl)-2-hydroxyéthylamino]propyl]phénoxy-méthyléthylphosphinate de lithium (RR) ;
4-[2-[2-(3,4-dihydroxyphényl)-2-hydroxyéthylamino]propyl]-phénoxyméthyléthylphosphinate d'éthyle (RR RS) ; et
acide (RR RS) 4-[2-[2-(3,4-dihydroxyphényl)-2-hydroxyéthylamino]propyl]-phénoxyméthylphénylphosphinique ; ou un des ses sels pharmaceutiquement acceptables, ou bien un de ses produits de solvatation pharmaceutiquement acceptables.

10. Procédé pour la préparation d'un composé de formule (I), ou d'un de ses sels d'addition d'acides pharmaceutiquement acceptables ou bien d'un de ses produits de solvatation pharmaceutiquement acceptables, procédé qui comprend :
(a) la réduction d'un composé de formule (II) : 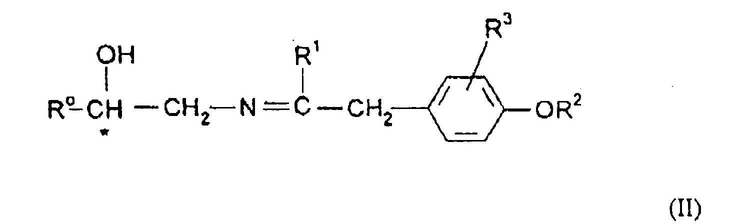 dans laquelle R⁰, R¹, R² et R³ répondent aux définitions mentionnées en rapport avec la formule (I) dans la revendication 1 ; ou
(b) la suppression de la production d'un composé de formule (IX) 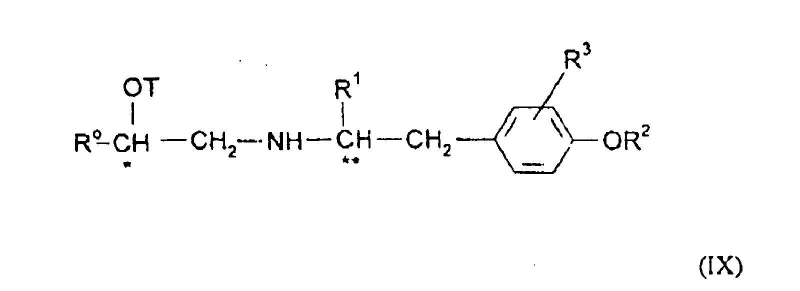 dans laquelle R⁰, R¹, R² et R³ répondent aux définitions mentionnées en rapport avec la formule (I) et T représente un groupe protecteur de la fonction hydroxy ;
et ensuite, si nécessaire, la mise en oeuvre d'une ou plusieurs des étapes facultatives suivantes :
(i) transformation d'un composé de formule (I) en un autre composé de formule (I) ; et
(ii) préparation d'un sel d'addition d'acide pharmaceutiquement acceptable d'un composé de formule (I) ou d'un produit de solvatation pharmaceutiquement acceptable d'un tel composé.

11. Composition pharmaceutique comprenant un composé de formule (I), ou un de ses sels pharmaceutiquement acceptables, ou bien un de ses produits de solvatation pharmaceutiquement acceptables, et un support pharmaceutiquement acceptable.

12. Composé de formule (I), ou un de ses sels pharmaceutiquement acceptables, ou bien un de ses produits de solvatation pharmaceutiquement acceptables, destiné à être utilisé comme substance thérapeutique active.

13. Composé de formule (I), ou un de ses sels pharmaceutiquement acceptables, ou bien un de ses produits de solvatation pharmaceutiquement acceptables, destiné à être utilisé dans le traitement de l'hyperglycémie, de l'obésité, de l'athérosclérose, de l'hyperinsulinémie, de troubles gastro-instestinaux, ou le traitement d'ulcérations gastro-intestinales.

14. Utilisation d'un composé de formule (I), ou d'un de ses sels pharmaceutiquement acceptables, ou bien d'un de ses produits de solvatation pharmaceutiquement acceptables, pour la production d'un médicament destiné au traitement de l'hyperglycémie, de l'obésité, l'athérosclérose, de l'hyperinsulinémie, de troubles gastro-instestinaux, ou le traitement d'ulcérations gastro-intestinales.

15. Procédé pour accroître le gain de poids et/ou améliorer l'efficacité d'utilisation de la nourriture et/ou augmenter la masse maigre de l'organisme du bétail, procédé qui comprend l'administration au bétail d'une quantité non toxique efficace d'un composé de formule (I) ou d'un de ses sels acceptables du point de vue vétérinaire, ou bien d'un de ses produits de solvatation acceptables du point de vue vétérinaire.

16. Formulation de prémélange acceptable du point de vue vétérinaire, comprenant un composé de formule (I) ou un de ses sels acceptables du point de vue vétérinaire, ou bien un de ses produits de solvatation acceptables du point de vue vétérinaire, en association avec un support acceptable du point de vue vétérinaire.

17. Composé, consistant en un intermédiaire :
(a) de formule (II) : 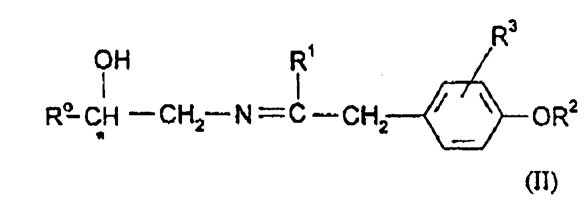 dans laquelle R⁰, R¹, R² et R³ répondent aux définitions mentionnées en rapport avec la formule (I) dans la revendication 1 ;
(b) de formule (IX) : 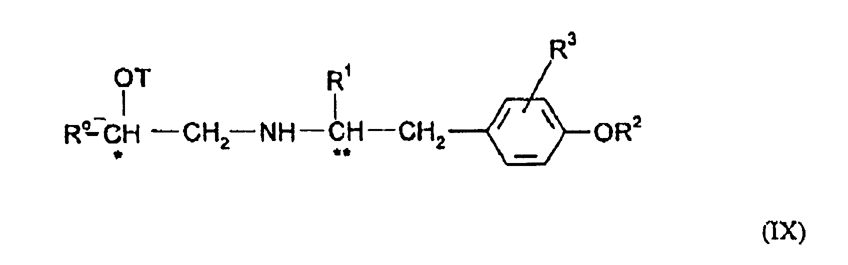 dans laquelle R⁰, R¹, R² et R³ répondent aux définitions mentionnées en rapport avec la formule (I) et T représente un groupe protecteur de la fonction hydroxy.
